# EUROPÄISCHE PATENTSCHRIFT

(11) **EP 0 667 334 B1**
(45) Veröffentlichungstag und Bekanntmachung des Hinweises auf die Patenterteilung: **07.01.1998**
(21) Anmeldenummer: 95101054.5
(22) Anmeldetag: 26.01.1995
(51) Int. Cl.: C07C 65/21, C07C 51/367, C07C 51/38

(54) **Verfahren zur Herstellung von 4-Alkoxy-3,5,6-trifluorphthalsäuren und 3-Alkoxy-2,4,5-trifluorbenzoesäuren**
Process for the preparation of 4-alkoxy-3,5,6-trifluorophthalic acids and 3-alkoxy-2,4,5-trifluorobenzoic acids
Procédé de préparation d'acides 4-alkoxy-3,5,6-trifluorophthaliques et d'acides 3-alkoxy-2,4,5-trifluorobenzoiques

(30) Priorität: 12.02.1994 DE 4404519
(43) Veröffentlichungstag der Anmeldung: 16.08.1995
(73) Patentinhaber: Clariant GmbH, 65929 Frankfurt am Main (DE)
(72) Erfinder: Pfirmann, Ralf, Dr., D-64347 Griesheim (DE); Papenfuhs, Theodor, Dr., D-60433 Frankfurt am Main (DE)

(56) Entgegenhaltungen:
- EP-A- 0 271 275
- CHEMICAL ABSTRACTS, vol. 116, no. 19, 1992, Columbus, Ohio, US; abstract no. 193915x, & JP-A-3 279 348 (KYORIN PHARMACEUTICAL CO., LTD) 10. Dezember 1991

## Beschreibung

Die vorliegende Erfindung betrifft ein neues, vorteilhaftes Verfahren zur Herstellung von 4-Alkoxy-3,5,6-trifluorphthalsäuren und gegebenenfalls 3-Alkoxy-2,4,5-trifluorbenzoesäuren.

Sowohl die 4-Alkoxy-3,5,6-trifluorphthalsäuren als auch die 3-Alkoxy-2,4,5-trifluorbenzoesäure stellen wichtige Zwischenprodukte für die Herstellung neuer hochwirksamer, antibakterieller Mittel aus der Reihe der Fluorchinoloncarbonsäuren (JP 63 297 366; EP-A-0 443 498; EP-A-0 230 295; EP-A-0 241 206 und JP 63 316 757) dar.

Die 4-Alkoxy-3,5,6-trifluorphthalsäuren sind im allgemeinen nur über einen aufwendigen und daher wenig ökonomischen Syntheseweg zugänglich. So läßt sich 4-Methoxy-3,5,6-trifluorphthalsäure auf folgende Weise (N. Ishikawa et al., Nippon Kagaku Kaishi 1 (1976), 200-202, JP-A-3279348 und EP-A-0 271 275) herstellen: Man setzt Tetrafluorphthalsäure mit Methanol zu dem entsprechenden Tetrafluorphthalsäuredimethylester um, führt anschließend unter Austausch des in der 4-Position befindlichen Fluoratoms eine Methoxygruppe ein und erhält auf diese Weise den 4-Methoxy-3,5,6-trifluorphthalsäuredimethylester. Der 4-Methoxv-3,5,6-trifluorphthalsäuredimethylester wird anschließend verseift, wobei die 4-Methoxy-3,5,6-trifluorphthalsäure gebildet wird. Die 4-Methoxy-3,5,6-trifluorphthalsäure läßt sich, falls gewünscht, durch Decarboxylierung in die 3-Methoxy-2,4,5-trifluorbenzoesäure überführen. Vergleiche auch nachstehendes Formelschema A.

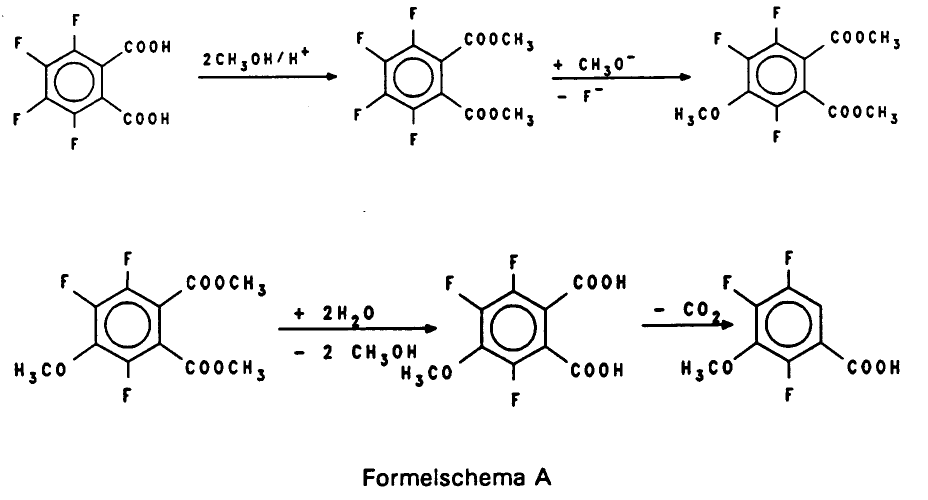

Andere technisch gangbare und bekannte Verfahren zur Herstellung von 3-Methoxy-2,4,5-trifluorbenzoesäure, 3-Ethoxy-2,4,5-trifluorbenzoesäure, 3-Propoxy-2,4,5-trifluorbenzoesäure und 3-Butoxy-2,4,5-trifluorbenzoesäure verlaufen stets über die vergleichsweise leicht zugängliche 3-Hydroxy-2,4,5-trifluorbenzoesäure, deren in 3-Stellung befindliche Hydroxy-Gruppe selektiv alkyliert werden muß (JP 01 268 662). Die 3-Hydroxy-2,4,5-trifluorbenzoesäure läßt sich nach EP 271275, wie nachfolgend erläutert, herstellen. Man setzt Tetrafluorphthalsäure unter den Bedingungen einer wäßrig-alkalischen Hydrolyse um, wobei durch den Austausch des in der 4-Position befindlichen Fluoratoms gegen eine Hydroxidgruppe die 4-Hydroxy-3,5,6-trifluorphthalsäure gebildet wird. Anschließend wandelt man die 4-Hydroxy-3,5,6-trifluorphthalsäure unter Decarboxylierung in die 3-Hydroxy-2,4,5-trifluorbenzoesäure um. Die 3-Hydroxy-2,4,5-trifluorbenzoesäure wird anschließend mit einem Alkylierungsmittel, beispielsweise Dimethylsulfat, in die entsprechende 3-Alkoxy-2,4,5-trifluorbenzoesäure, bzw. in die 3-Methoxy-2,4,5-trifluorbenzoesäure umgesetzt.

Setzt man entsprechend diesem Verfahren 4-Alkoxy-3,5,6-trifluorphthalsäuren anstelle der 4-Hydroxy-3,5,6-trifluorphthalsäure in einem wäßrigen sauren Medium zur Decarboxylierung ein, so bilden sich die 3-Alkoxy-2,4,5-Trifluorbenzoesäuren nicht oder lediglich in beschränktem Umfange. Die Ursache hierfür ist, daß unter den Bedingungen der Decarboxylierung gleichzeitig eine Etherspaltung abläuft, wobei aus den 4-Alkoxy-3,5,6-trifluorphthalsäuren jeweils die 3-Hydroxy-2,4,5-trifluorbenzoesäure, nicht jedoch die entsprechende 3-Alkoxy-2,4,5-trifluorbenzoesäure, gebildet wird (EP 271275). Der vorstehend beschriebene Syntheseweg läßt sich dem nachfolgenden Formelschema B entnehmen:

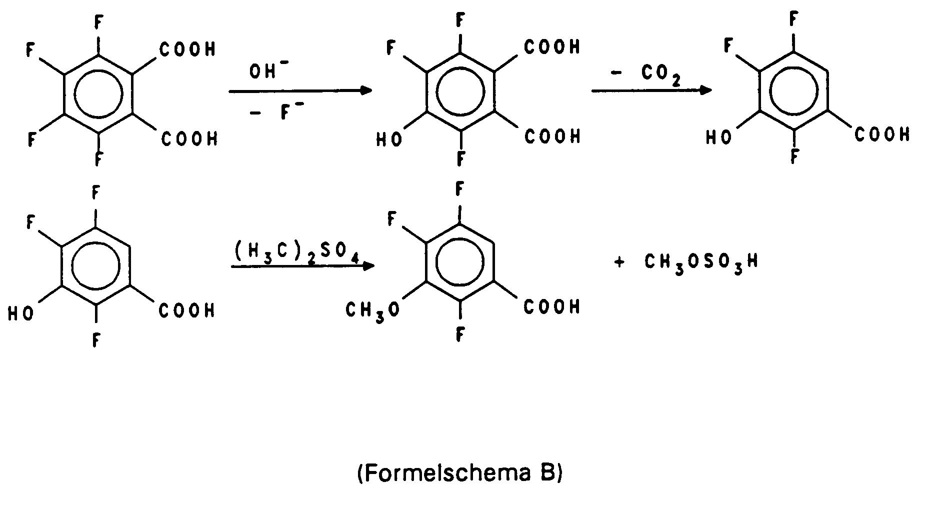

Im Gegensatz zu den in Formelschema B beschriebenen Verfahren liefert das Verfahren gemäß Formelschema A zwar auch 4-Alkoxy-3,5,6-trifluorphthalsäuren, das Verfahren erweist sich jedoch wegen der Anzahl der Verfahrensschritte als umständlich. Zudem erfordert es aufgrund der Stöchiometrie der Umsetzung nicht weniger als 3 Mole Alkohol je Mol herzustellender 4-Alkoxy-3,5,6-trifluorphthalsäure bzw. 3-Alkoxy-2,4,5-trifluorbenzoesäure. Nicht weniger als 2 Mol Alkohol werden benötigt, um aus der Tetrafluorphthalsäure die entsprechenden Diester herzustellen. Die beiden Estergruppen dienen lediglich als Schutzgruppe und werden anschließend wieder unter Abspaltung von Alkohol hydrolytisch gespalten. Dies ist im Hinblick auf schwer zugängliche, wertvolle Alkohole zusätzlich von Nachteil, da es nicht als ökonomisch anzusehen ist, aus wertvollen Alkoholen aufgebaute Estergruppen anschließend wieder zu hydrolysieren.

Das Verfahren gemäß Formelschema B besitzt im Hinblick auf den Einbau von Alkoxy-Gruppen eine relativ geringe Variabilität, da die für die Synthese benötigten, die entsprechenden für die Herstellung der Wirkstoffe interessanten Alkylgruppen enthaltenden Alkylierungsmittel lediglich in einer stark begrenzten Anzahl zur Verfügung stehen. Zudem ist die Handhabung von Alkylierungsmitteln, beispielsweise Alkylhalogeniden oder Dialkylsulfaten, recht problematisch, da diese Stoffe sehr toxisch sind.

Der vorliegenden Erfindung liegt somit die Aufgabe zugrunde, ein gegenüber dem Stand der Technik vereinfachtes Verfahren zur Herstellung nicht nur von 4-Alkoxy-3,5,6-trifluorphthalsäuren sondern auch, falls gewünscht, von 3-Alkoxy-2,4,5-trifluorbenzoesäuren bereitzustellen, das die vorstehend genannten Nachteile nicht aufweist und sich zudem in möglichst großer Breite anwenden läßt.

Gelöst wird diese Aufgabe durch ein Verfahren zur Herstellung von 4-Alkoxy-3,5,6-trifluorphthalsäuren der allgemeinen Formel 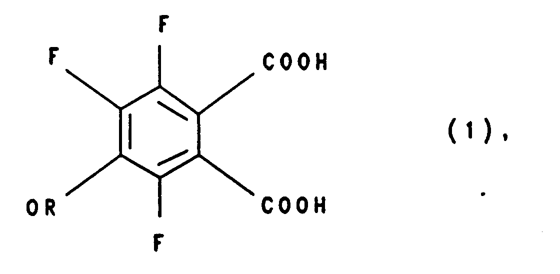 worin R für einen gegebenenfalls einfach oder mehrfach fluorierten Alkylrest mit 1 bis 5 Kohlenstoffatomen, für einen gegebenenfalls einfach oder mehrfach fluorierten Cycloalkylrest, mit 3 bis 5 Kohlenstoffatomen im Ring oder für einen gegebenenfalls einfach oder mehrfach fluorierten araliphatischen Rest steht, und gegebenenfalls von 3-Alkoxy-2,4,5-trifluorbenzoesäuren der allgemeinen Formel 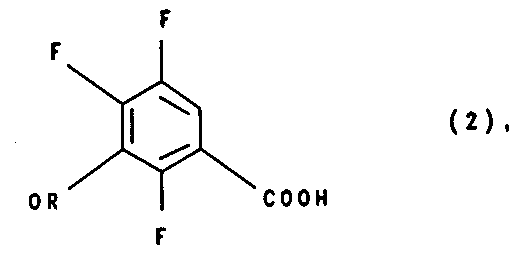 worin R die vorstehende Bedeutung hat. Es ist dadurch gekennzeichnet, daß man Tetrafluorphthalsäure oder Tetrafluorphthalsäureanhydrid mit einem Alkohol der allgemeinen Formel ROH, worin R die vorstehende Bedeutung hat, und einer wasserlöslichen Base in Wasser bei erhöhter Temperatur umsetzt, die 4-Alkoxy-3,5,6-trifluorphthalsäure isoliert und gegebenenfalls die 4-Alkoxy-3,5,6-trifluorphthalsäure in Anwesenheit eines organischen basischen Lösungsmittels und gegebenenfalls eines inerten Lösungsmittels bei 70 bis 180 °C decarboxyliert und die gebildete 3-Alkoxy-2,4,5-trifluorbenzoesäure isoliert.

Die dem erfindungsgemäßen Verfahren zugrundeliegende Reaktionsfolge ist dem nachfolgenden Formelschema C zu entnehmen.

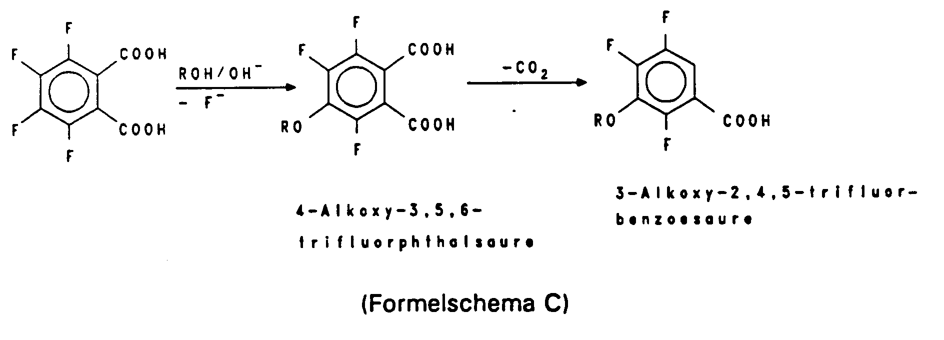

Wie ein einfacher Vergleich mit den Reaktionsfolgen der Formelschemata A und B zeigt, führt das erfindungsgemäße Verfahren gegenüber dem Stand der Technik zu einer deutlichen Vereinfachung. Es läßt sich zudem in einem breiten Umfange anwenden, da sich für den ersten Reaktionsschritt, nämlich den Austausch des in 4-Position befindlichen Fluoratoms gegen eine Alkoxygruppe eine Vielzahl verschiedener Alkohole eignet. Zudem wird für die Herstellung von einem Mol 4-Alkoxy-3,5,6-trifluorphthalsäure bzw. 3-Alkoxy-2,4,5-trifluorbenzoesäure lediglich ein Mol Alkohol verbraucht. Überraschenderweise ist es nicht erforderlich, die beiden Carboxylgruppen der Tetrafluorphthalsäure durch Veresterung zu schützen. Die Umsetzung verläuft mit hoher Selektivität und führt zu einer hohen Ausbeute an 4-Alkoxy-3,5,6 trifluorphthalsäure. Darüber hinaus stellt die vorliegende Erfindung sicher, daß auch der zweite Reaktionsschritt, nämlich die Decarboxylierung der 4-Alkoxy-3,5,6-trifluorphthalsäure zur 3-Alkoxy-2,4,5-trifluorbenzoesäure mit hoher Ausbeute abläuft.

Es ist ferner als überraschend anzusehen, daß die Bildung von Nebenprodukten, insbesondere die Etherspaltung, die zur Bildung von 3-Hydroxy-2,4,5-trifluorbenzoesäure führt, vollständig vermieden wird.

Zur Umsetzung von Tetrafluorphthalsäure oder Tetrafluorphthalsäureanhydrid eignen sich, ohne Anspruch auf Vollständigkeit zur erheben, geradkettige oder verzweigte aliphatische Alkohole, cycloaliphatische Alkohole und araliphatische Alkohole. In die Reaktion läßt sich als Alkohol der allgemeinen Formel ROH ein primärer oder sekundärer Alkohol, insbesondere ein primärer Alkohol einsetzen.

Als Beispiel für Alkohole der Formel ROH seien Methanol, Ethanol, n-Propanol, n-Butanol, n-Pentanol, Cyclopropanol, Hydroxymethylcyclopropan, Fluormethanol, Difluormethanol, Trifluormethanol, Monofluorethanol, Difluorethanol, Trifluorethanol, Monofluorpropanol, Monofluorbutanol, Monofluorcyclopropanol, Difluorcyclopropanol, Benzylalkohol, 4-Fluorbenzylalkohol oder Phenylethanol, insbesondere Methanol, Ethanol, Cyclopropanol, n-Propanol, Fluormethanol, Difluormethanol, Trifluormethanol, Monofluorethanol, Monofluorbutanol, Benzylalkohol oder 4-Fluorbenzylalkohol genannt.

Zur Durchführung der Umsetzung empfiehlt es sich, 1 bis 100, insbesondere 20 bis 80, bevorzugt 40 bis 60 Mol Alkohol je Mol Tetrafluorphthalsäure oder Tetrafluorphthalsäureanhydrid einzusetzen.

Die Umsetzung läuft in Gegenwart einer wasserlöslichen Base in Wasser ab. Man setzt ein Hydroxid, Carbonat, Phosphat, Hydrogenphosphat oder Dihydrogenphosphat eines Alkalimetalls oder Erdalkalimetalls oder Mischungen derselben als wasserlösliche Base ein. Gut geeignet als wasserlösliche Base sind Lithiumhydroxid, Lithiumhydroxidhydrat, Natriumhydroxid oder Kaliumhydroxid.

Üblicherweise setzt man 1 bis 100, insbesondere 1,1 bis 10, bevorzugt 2 bis 5 Mol Base je Mol Tetrafluorphthalsäure oder Tetrafluorphthalsäureanhydrid ein.

Die Reaktion läuft in Anwesenheit von Wasser. Üblicherweise setzt man 1 bis 1500, insbesondere 10 bis 300, bevorzugt 25 bis 200 Mol Wasser je Mol Tetrafluorphthalsäure oder Tetrafluorphthalsäureanhydrid ein.

Die Umsetzung von Tetrafluorphthalsäure oder Tetrafluorphthalsäureanhydrid mit dem Alkohol der Formel ROH und der wasserlöslichen Base in Wasser erfolgt bei erhöhter Temperatur, üblicherweise bei 25 bis 150 °C. In einer Vielzahl von Fällen hat es sich als ausreichend erwiesen, die Umsetzung bei 50 bis 120, insbesondere 60 bis 100 °C ablaufen zu lassen. Es sei an dieser Stelle jedoch darauf hingewiesen, daß die zu wählende Reaktionstemperatur in gewissem Umfange von der Reaktivität des jeweils eingesetzten Alkoholes und den übrigen Reaktionsbedingungen abhängig ist. Sie ist den jeweiligen Gegebenheiten entsprechend anzupassen.

Nach Beendigung der Reaktion erhält man ein wäßriges Reaktionsgemisch, aus dem das jeweils gewünschte Wertprodukt abgetrennt werden muß. Üblicherweise destilliert man aus dem wäßrigen Reaktionsgemisch nichtumgesetzten Alkohol ab und isoliert die 4-Alkoxy-3,5,6-trifluorphthalsäure durch Zusatz von Säure und/oder Extraktion aus dem wäßrigen Reaktionsgemisch.
Man extrahiert das von nichtumgesetztem Alkohol befreite wäßrige Reaktionsgemisch mit einem in Wasser unlöslichen organischen Solvens oder dem basischen Lösungsmittel. Geeignet als wasserunlösliches organisches Solvens sind polare organische Lösungsmittel, beispielsweise Dialkylether oder Ester aliphatischer Carbonsäuren mit 1 bis 6 Kohlenstoffatomen.

Beabsichtigt man die gebildete 4-Alkoxy-3,5,6-trifluorphthalsäure durch Decarboxylierung in die 3-Alkoxy-2,4,5-trifluorbenzoesäure umzuwandeln, so empfiehlt es sich, das wäßrige, von nichtumgesetztem Alkohol befreite Reaktionsgemisch mit dem basischen Lösungsmittel zu extrahieren und dieses Gemisch für die weitere Verarbeitung zu benutzen. Diese Arbeitsweise gestaltet sich besonders einfach.

Man setzt als basisches Lösungsmittel eine organische Base, beispielsweise ein geradkettiges oder verzweigtes aliphatisches Amin, ein oder mehrfach substituiertes Pyrrol, Pyrrolidon, Imidazol, Imidazolidinon, Pyridin, Pyrimidin, Chinolin, Isochinolin, ein N,N-dialkyliertes Carbonsäureamid oder Gemische dieser Verbindungen, insbesondere ein Trialkylamin mit 4 bis 20, vorzugsweise 6 bis 14 Kohlenstoffatomen im Alkylrest, Gemische dieser Trialkylamine, Collidin, Chinolin, N-Methylpyrrolidon, N,N-Dimethylacetamid, 1,3-Dimethylimidazolidinon oder Gemische dieser Verbindungen, ein.

In einer Reihe von Fällen kann es nützlich sein, zusätzlich ein unter den Bedingungen der Umsetzung inertes Lösungmittel zu verwenden. Man setzt als inertes Lösungsmittel aromatische Kohlenwasserstoffe, einfach oder mehrfach halogenierte aromatische Kohlenwasserstoffe oder Gemische derselben ein. Ohne den Anspruch auf Vollständigkeit zu erheben, seien als geeignete inerte Lösungsmittel Toluol, Ethylbenzol, Mesitylen, o-Xylol, m-Xylol, p-Xylol, technische Gemische isomerer Xylole, Chlorbenzol, Dichlorbenzol, Trichlorbenzol, Chlortoluol, Dichlortoluol oder Gemische derselben genannt.

Wie zuvor bereits erwähnt, führt man die Decarboxylierung bei 70 bis 180 °C durch. In einer Vielzahl von Fällen hat es sich als ausreichend erwiesen, die Decarboxylierung bei 80 bis 170, insbesondere 100 bis 150 °C durchzuführen.

Nach Decarboxylierung extrahiert man das Reaktionsgemisch mit einer wäßrigen Lösung mit einer Base, separiert die dabei anfallende wäßrige Phase, fällt die 3-Alkoxy-2,4,5-trifluorbenzoesäure durch Ansäuern aus und isoliert sie durch Filtration. Als Base lassen sich Alkalihydroxide, Alkalicarbonate, insbesondere Lithiumhydroxid, Natriumhydroxid, Kaliumhydroxid gelöst in Wasser verwenden. Nach Abtrennung der wäßrigen Phase setzt man üblicherweise eine Mineralsäure, beispielsweise Salzsäure, Schwefelsäure, Phosphorsäure, in ausreichender Menge, d.h. bis zu einem pH-Wert von 0 bis 3, insbesondere 1 bis 2,5 zu und filtriert die 3-Alkoxy-2,4,5-trifluorbenzoesäure ab.

Die nachfolgenden Beispiele beschreiben die Erfindung, ohne sie zu beschränken.

### Beispiel 1

### Umsetzung mit Methanol

Man löst 4,4 g (20 mMol) Tetrafluorphthalsäureanhydrid in 6 g Wasser und 30 g Methanol. Dabei steigt die Temperatur auf 30 °C und die Lösung nimmt eine gelbe Färbung an. Man gibt 8,2 g (0,2 Mol) Lithium-hydroxidhydrat zu und erhitzt 10 Stunden am Rückfluß (65 °C). Danach läßt sich mittels GC-Analyse kein Tetrafluorphthalsäureanhydrid mehr nachweisen. Man setzt 30 g Wasser zu und destilliert bis zu einer Kopftemperatur von 100 °C nichtumgesetztes Methanol (39 g) vollständig ab. Danach stellt man durch Zugabe von 30 %iger Salzsäure einen pH-Wert von 1 ein und saugt den anfallenden farblosen Niederschlag ab. Man extrahiert die wässrige Phase zweimal mit einem Gemisch aus je 20 g 1,2-Dichlorbenzol und 10 g Hostarex A327 (ein Gemisch verschiedener aliphatischer Trialkylamine mit 6 bis 14 Kohlenstoffatomen im Alkylrest). Danach enthält die wäßrige Phase keinerlei durch GC-Analyse nachweisbare organische Säuren. Die Extraktphase wird 2 Stunden auf 140 °C bis zur Beendigung der Gasentwicklung (Decarboxylierung) erhitzt. Nach dem Abkühlen wird mit Natronlauge alkalisch gestellt (pH = > 11) und mehrfach mit Dichlormethan extrahiert. Die verbleibende wäßrige Phase wird weiterverarbeitet, indem das Hostarex A327 sowie dir Lösungsmittel destillativ zurückgewonnen werden. Man säuert anschließend die wäßrige Phase erneut an (pH = 1) und extrahiert zweimal mittels je 20 g Methyl-tert.butylether (MTBE). Der Extrakt wird über MgSO₄ getrocknet, filtriert und das Lösungsmittel im Rotationsverdampfer entfernt. Es verbleiben 2,7 (13,1 mMol, entsprechend 66 %) leicht gelblich gefärbtes Pulver, das gemäß GC-MS als Methoxy-trifluorbenzoesäure (Reingehalt GC > 95 %) ausgewiesen wird. Die weitere Reinigung kann durch Ausrühren (Umkristallisieren) aus heißem Wasser erfolgen.

### Beispiel 2

### Umsetzung mit Methanol

Man verfährt wie in Beispiel 1, extrahiert jedoch nicht mittels Hostarex A327, sondern extrahiert stattdessen dreimal mit je 20 g MTBE. Nach Trocknen der organischen Phase über MgSO₄, Filtration und Entfernen des Lösungsmittels verbleiben 3,8 g (15,2 mMol, entsprechend 76 %) gelbliche 4-Methoxy-3,5,6-trifluorphthalsäure (Reingehalt gemäß GC > 90 %).

### Beispiel 3

### Umsetzung mit Ethanol

4,4 g (20 mMol) Tetrafluorphthalsäureanhydrid werden in 3 g Wasser und 30 g Ethanol gelöst und mit 4,2 g Lithiumhydroxidhydrat versetzt und 48 Stunden am Rückfluß erhitzt. Man gibt anschließend 50 g Wasser zu und destilliert solange ein Ethanol-Wasser-Gemisch (31,9 g) ab, bis eine Kopftemperatur von 100 °C erreicht wird. Danach stellt man durch Zugabe von 19 g einer 30 %igen Salzsäure einen pH-Wert von 0,6 ein und extrahiert mit einem Gemisch aus 20 g 1,2-Dichlorbenzol und 10 g Hostarex A327. Die über MgSO₄ getrocknete organische Phase wird eine Stunde auf 140 °C bis zur Beendigung der Gasentwicklung (Decarboxylierung) erhitzt und anschließend bei 25 °C mit 12,1 g 35 %iger Natronlauge auf pH = 14 eingestellt. Man extrahiert dreimal mit je 50 g Dichlormethan, stellt die wäßrige Phase durch Zugabe von 9,4 g 30 %iger Salzsäure auf pH = 0,7 und extrahiert mit MTBE. Nach Trocknen über MgSO₄, Filtration und Entfernen des Lösungsmittels verbleiben 3,4 g (15,5 mMol, entsprechend 77 %) gelbliche 3-Ethoxy-2,4,5-trifluorbenzoesäure (Reingehalt gemäß GC > 95 %).

### 3-Ethoxy-2,4,5-trifluorbenzoesäure:

- MS: m/z (%):: 99 (8,8), 119 (7,8), 147 (6,5), 174 (5,1), 175 (98,2), 192 (100), 193 (6,2), 220 (23,4)

### Beispiel 4

### Umsetzung mit Ethanol

Verfährt man wie in Beispiel 3 angegeben, isoliert hingegen die 4-Ethoxy-3,5,6-trifluorphthalsäure wie in Beispiel 2 beschrieben, so erhält man 4,6 g (17,4 mMol, entsprechend 87 %) 4-Ethoxy-3,5,6-trifluorphthalsäure.

### 4-Ethoxy-3,5,6-trifluorphthalsäure:

- MS: m/z (%):: 78 (9,8), 98 (26,3), 117 (29), 146 (71,4), 174 (100), 175 (5,9), 218 (70,6), 246 (25,5, (M-H₂O)⁺

### Beispiel 5

### Umsetzung mit Trifluorethanol

Man legt 7,6 g Wasser 2,1 g Lithiumhydroxid-Hydrat 10 g Trifluorethanol und 2,2 g (10 mMol) Tetrafluorphthalsäureanhydrid vor. Die Mischung wird 96 Stunden auf 70 °C erwärmt, nach dieser Zeit ist der Gehalt an Tetrafluorphtalsäureanhydrid unter 1 % (GC) gesunken. Man verfährt weiter, wie in Beispiel 3 beschrieben, und erhält 1,4g (5,1 mMol, entsprechend 51 %) 3-Trifluorethoxy-2,4,5-trifluorbenzoesäure.

### Beispiel 6

### Umsetzung mit Trifluorethanol

Verfährt man nach Beispiel 5, verzichtet jedoch auf die Decarboxylierung und isoliert die Phthalsäure, wie in Beispiel 2 beschrieben, so erhält man 2,2 g (6,9 mMol, entsprechend 69 %) 4-Trifluorethoxy-3,5,6-trifluorphthalsäure als leicht gelblichen Feststoff.

Dieser kann, durch Aufnehmen in Hostarex A 327 (324) und Erhitzen decarboxyliert werden, wobei weiter, wie in Beispiel 5 angegeben, verfahren wird. Vollständig analog läßt sich 2,2,3,3-Tetrafluorpropanol umsetzen.

### 4-Trifluorethoxy-3,5,6-trifluorphthalsäure:

- MS: m/z [%]:: 79 (14), 83 (7), 98 (12), 117 (42), 129 (14), 145 (17), 146 (11), 157 (5), 159 (30), 228 (100), 229 (8), 256 (89), 281 (13), 300 (M-H₂O)^{⊕}, 90), 301 (9)

### Beispiel 7

### Umsetzung mit 4-Fluorbenzylalkohol

In 30 g Wasser werden 8,4 g (0,15 Mol) Kaliumhydroxid gelöst, 6,6 g (30 mMol) Tetrafluorphthalsäureanhydrid und 30 g 4-Fluorbenzylalkohol (0,238 Mol) zugegeben. Man erhitzt 12 Stunden auf 100 °C und verfährt weiter, wie in Beispiel 2 beschrieben. Es ist von Vorteil, den pH-Wert vor den Extraktionsschritten nicht unter 3 fallen zu lassen. Nach dieser Methode erhält man 4,2 g (14 mMol, entsprechend 47 %) (4-Fluorbenzyloxy)trifluorbenzoesäure (Reingehalt 80 - 85 %).

## Patentansprüche

1. Verfahren zur Herstellung von 4-Alkoxy-3,5,6-trifluorphthalsäuren der allgemeinen Formel 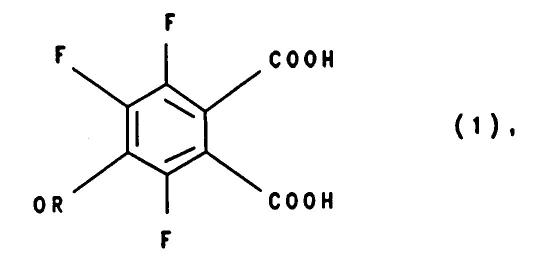 worin R für einen gegebenenfalls einfach oder mehrfach fluorierten Alkylrest mit 1 bis 5 Kohlenstoffatomen, für einen gegebenenfalls einfach oder mehrfach fluorierten Cycloalkylrest mit 3 bis 5 Kohlenstoffatomen im Ring oder für einen gegebenenfalls einfach oder mehrfach fluorierten araliphatischen Rest steht, und gegebenenfalls von 3-Alkoxy-2,4,5-trifluorbenzoesäure der allgemeinen Formel 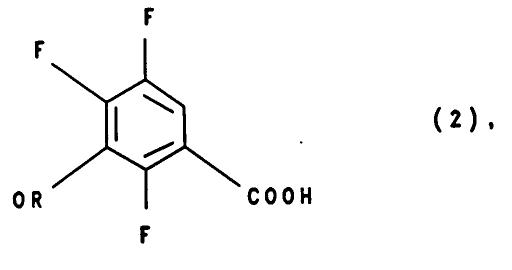 worin R die vorstehende Bedeutung hat, dadurch gekennzeichnet, daß man Tetrafluorphthalsäure oder Tetrafluorphthalsäureanhydrid mit einem Alkohol der allgemeinen Formel ROH, worin R die vorstehende Bedeutung hat, und einer wasserlöslichen Base in Wasser bei erhöhter Temperatur umsetzt, die gebildete 4-Alkoxy-3,5,6-trifluorphthalsäure isoliert und gegebenenfalls die 4-Alkoxy-3,5,6-trifluorphthalsäure in Anwesenheit eines organischen basischen Lösungsmittels und gegebenenfalls eines inerten Lösungsmittels bei 70 bis 180 °C decarboxyliert und die gebildete 3-Alkoxy-2,4,5-trifluorbenzoesäure isoliert.

2. Verfahren nach Anspruch 1, dadurch gekennzeichnet, daß man als Alkohol der allgemeinen Formel ROH einen primären Alkohol einsetzt.

3. Verfahren nach Anspruch 1 oder 2, dadurch gekennzeichnet, daß man als Alkohol der Formel ROH Methanol, Ethanol, n-Propanol, n-Butanol, n-Pentanol, Cyclopropanol, Hydroxymethylcyclopropan, Fluormethanol, Difluormethanol, Trifluormethanol, Monofluorethanol, Difluorethanol, Trifluorethanol, Monofluorpropanol, Monofluorbutanol, Monofluorcyclopropanol, Difluorcyclopropanol, Benzylalkohol, 4-Fluorbenzylalkohol oder Phenylethanol einsetzt.

4. Verfahren nach einem oder mehreren der Ansprüche 1 bis 3, dadurch gekennzeichnet, daß man als Alkohol der Formel ROH Methanol, Ethanol, Cyclopropanol, n-Propanol, Fluormethanol, Difluormethanol, Trifluormethanol, Monofluorethanol, Monofluorbutanol, Benzylalkohol oder 4-Fluorbenzylalkohol einsetzt.

5. Verfahren nach einem oder mehreren der Ansprüche 1 bis 4, dadurch gekennzeichnet, daß man 1 bis 100, insbesondere 20 bis 80, bevorzugt 40 bis 60 Mol Alkohol je Mol Tetrafluorphthalsäure oder Tetrafluorphthalsäureanhydrid einsetzt.

6. Verfahren nach einem oder mehreren der Ansprüche 1 bis 5, dadurch gekennzeichnet, daß man ein Hydroxid, Carbonat, Phosphat, Hydrogenphosphat oder Dihydrogenphosphat eines Alkalimetalls oder Erdalkalimetalls oder Mischungen derselben als wasserlösliche Base einsetzt.

7. Verfahren nach einem oder mehreren der Ansprüche 1 bis 6, dadurch gekennzeichnet, daß man Lithiumhydroxid, Lithiumhydroxidhydrat, Natriumhydroxid oder Kaliumhydroxid als wasserlösliche Base einsetzt.

8. Verfahren nach einem oder mehreren der Ansprüche 1 bis 7, dadurch gekennzeichnet, daß man 1 bis 100, insbesondere 1,1 bis 10, bevorzugt 2 bis 5 Mol Base, je Mol Tetrafluorphthalsäure oder Tetrafluorphthalsäureanyhdrid einsetzt.

9. Verfahren nach einem oder mehreren der Ansprüche 1 bis 8, dadurch gekennzeichnet, daß man 1 bis 1500, insbesondere 10 bis 300, bevorzugt 25 bis 200 Mol Wasser je Mol Tetrafluorphthalsäure oder Tetrafluorphthalsäureanyhdrid einsetzt.

10. Verfahren nach einem oder mehreren der Ansprüche 1 bis 9, dadurch gekennzeichnet, daß man die Tetrafluorphthalsäure oder das Tetrafluorphthalsäureanhydrid mit dem Alkohol bei 25 bis 150, insbesondere 50 bis 120 bevorzugt 60 bis 100 °C umsetzt.

11. Verfahren nach einem oder mehreren der Ansprüche 1 bis 10, dadurch gekennzeichnet, daß man aus dem wäßrigen Reaktionsgemisch nicht umgesetzten Alkohol abdestilliert, die 4-Alkoxy-3,5,6-trifluorphthalsäure durch Zusatz von Säure und/oder durch Extraktion aus dem wäßrigen Reaktionsgemisch isoliert.

12. Verfahren nach einem oder mehreren der Ansprüche 1 bis 11, dadurch gekennzeichnet, daß man das wäßrige Reaktionsgemisch mit einem in Wasser unlöslichen organischen Solvens, insbesondere einem Dialkylether oder einem Ester einer aliphatischen Carbonsäure mit 1 bis 6 Kohlenstoffatomen, oder dem basischen Lösungsmittel extrahiert.

13. Verfahren nach einem oder mehreren der Ansprüche 1 bis 12, dadurch gekennzeichnet, daß man ein geradkettiges oder verzweigtes aliphatisches Amin, ein gegebenenfalls einfach oder mehrfach alkylsubstituiertes Pyrrol, Pyrrolidon, Imidazol, Imidazolidinon, Pyridin, Pyrimidin, Chinolin, Isochinolin, ein N,N-dialkyliertes Carbonsäureamid oder Gemische dieser Verbindungen als organisches basisches Lösungsmittel einsetzt.

14. Verfahren nach einem oder mehreren der Ansprüche 1 bis 13, dadurch gekennzeichnet, daß man ein Trialkylamin mit 4 bis 20 Kohlenstoffatomen im Alkylrest, Gemische dieser Trialkylamine, Collidin, Chinolin, N-Methylpyrrolidon, N,N-Dimethylacetamid, 1,3-Dimethylimidazolidinon oder Gemische dieser Verbindungen als basisches Lösungsmittel einsetzt.

15. Verfahren nach einem oder mehreren der Ansprüche 1 bis 14, dadurch gekennzeichnet, daß man aromatische Kohlenwasserstoffe, einfach oder mehrfach halogenierte aromatische Kohlenwasserstoffe oder Gemische derselben als inertes Lösungsmittel einsetzt.

16. Verfahren nach einem oder mehreren der Ansprüche 1 bis 15, dadurch gekennzeichnet, daß man Toluol, Ethylbenzol, Mesitylen, o-Xylol, m-Xylol, p-Xylol, technische Gemische isomerer Xylole, Chlorbenzol, Dichlorbenzol, Trichlorbenzol, Chlortoluol, Dichlortoluol oder Gemische derselben als inertes Lösungsmittel einsetzt.

17. Verfahren nach einem oder mehreren der Ansprüche 1 bis 16, dadurch gekennzeichnet, daß man bei 80 bis 170, insbesondere 100 bis 150 °C decarboxyliert.

18. Verfahren nach einem oder mehreren der Ansprüche 1 bis 17, dadurch gekennzeichnet, daß man das Reaktionsgemisch nach Decarboxylierung mit einer wäßrigen Lösung einer Base extrahiert, die wäßrige Phase separiert, die 3-Alkoxy-2,4,5-trifluorbenzoesäure durch Ansäuern ausfällt und durch Filtration isoliert.

## Claims

1. A process for the preparation of a 4-alkoxy-3,5,6-trifluorophthalic acid of the formula 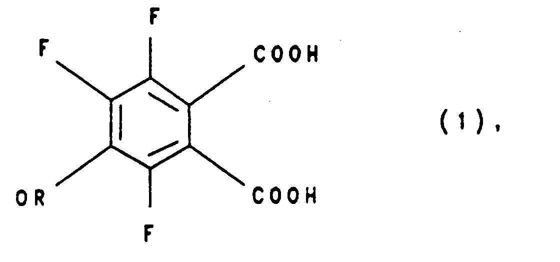 in which R is an alkyl radical having 1 to 5 carbon atoms which may be monofluorinated or polyfluorinated, a cycloalkyl radical having 3 to 5 carbon atoms in the ring which may be monofluorinated or polyfluorinated, or an araliphatic radical which may be monofluorinated or polyfluorinated, or, if desired, of a 3-alkoxy-2,4,5-trifluorobenzoic acid of the formula 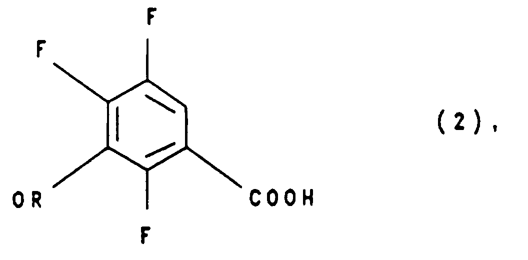 in which R is as defined above, which comprises reacting tetrafluorophthalic acid or tetrafluorophthalic anhydride with an alcohol of the formula ROH in which R is as defined above and with a water-soluble base in water at elevated temperature, isolating the 4-alkoxy-3,5,6-trifluorophthalic acid formed and, if desired, decarboxylating the 4-alkoxy-3,5,6-trifluorophthalic acid in the presence of an organic basic solvent and, if desired, of an inert solvent at from 70 to 180°C, and isolating the 3-alkoxy-2,4,5-trifluorobenzoic acid formed.

2. The process as claimed in claim 1, wherein the alcohol of the formula ROH employed is a primary alcohol.

3. The process as claimed in claim 1 or 2, wherein the alcohol of the formula ROH employed is methanol, ethanol, n-propanol, n-butanol, n-pentanol, cyclopropanol, hydroxymethylcyclopropane, fluoromethanol, difluoromethanol, trifluoromethanol, monofluoroethanol, difluoroethanol, trifluoroethanol, monofluoropropanol, monofluorobutanol, monofluorocyclopropanol, difluorocyclopropanol, benzyl alcohol, 4-fluorobenzyl alcohol or phenylethanol.

4. The process as claimed in one or more of claims 1 to 3, wherein the alcohol of the formula ROH employed is methanol, ethanol, cyclopropanol, n-propanol, fluoromethanol, difluoromethanol, trifluoromethanol, monofluoroethanol, monofluorobutanol, benzyl alcohol or 4-fluorobenzyl alcohol.

5. The process as claimed in one or more of claims 1 to 4, wherein from 1 to 100 mol, in particular from 20 to 80 mol and preferably from 40 to 60 mol of alcohol are employed per mole of tetrafluorophthalic acid or tetrafluorophthalic anhydride.

6. The process as claimed in one or more of claims 1 to 5, wherein a hydroxide, carbonate, phosphate, hydrogen phosphate or dihydrogen phosphate of an alkali metal or of an alkaline earth metal or a mixture thereof is employed as water-soluble base.

7. The process as claimed in one or more of claims 1 to 6, wherein lithium hydroxide, lithium hydroxide hydrate, sodium hydroxide or potassium hydroxide is employed as water-soluble base.

8. The process as claimed in one or more of claims 1 to 7, wherein from 1 to 100 mol, in particular from 1.1 to 10 mol and preferably from 2 to 5 mol of base are employed per mole of tetrafluorophthalic acid or tetrafluorophthalic anhydride.

9. The process as claimed in one or more of claims 1 to 8, wherein from 1 to 1500 mol, in particular from 10 to 300 mol and preferably from 25 to 200 mol of water are employed per mole of tetrafluorophthalic acid or tetrafluorophthalic anhydride.

10. The process as claimed in one or more of claims 1 to 9, wherein the tetrafluorophthalic acid or the tetrafluorophthalic anhydride is reacted with the alcohol at from 25 to 150°C, in particular from 50 to 120°C and preferably from 60 to 100°C.

11. The process as claimed in one or more of claims 1 to 10, wherein unreacted alcohol is distilled off from the aqueous reaction mixture and the 4-alkoxy-3,5,6-trifluorophthalic acid is isolated by addition of acid and/or by extraction from the aqueous reaction mixture.

12. The process as claimed in one or more of claims 1 to 11, wherein the aqueous reaction mixture is extracted with a water-insoluble organic solvent, in particular a dialkyl ether or an ester of an aliphatic carboxylic acid having 1 to 6 carbon atoms, or with a basic solvent.

13. The process as claimed in one or more of claims 1 to 12, wherein a straight-chain or branched aliphatic amine, an optionally mono- or polyalkyl-substituted pyrrole, pyrrolidone, imidazole, imidazolidinone, pyridine, pyrimidine, quinoline, isoquinoline, an N,N-dialkylated carboxamide or a mixture of these compounds is employed as organic basic solvent.

14. The process as claimed in one or more of claims 1 to 13, wherein a trialkylamine having 4 to 20 carbon atoms in the alkyl radical, a mixture of these trialkylamines, collidine, quinoline, N-methylpyrrolidone, N,N-dimethylacetamide, 1,3-dimethylimidazolidinone or a mixture of these compounds is employed as basic solvent.

15. The process as claimed in one or more of claims 1 to 14, wherein an aromatic hydrocarbon, a mono- or polyhalogenated aromatic hydrocarbon or a mixture thereof is employed as inert solvent.

16. The process as claimed in one or more of claims 1 to 15, wherein toluene, ethylbenzene, mesitylene, o-xylene, m-xylene, p-xylene, a technical-grade mixture of isomeric xylenes, chlorobenzene, dichlorobenzene, trichlorobenzene, chlorotoluene, dichlorotoluene or a mixture thereof is employed as inert solvent.

17. The process as claimed in one or more of claims 1 to 16, wherein decarboxylation is carried out at from 80 to 170°C, in particular from 100 to 150°C.

18. The process as claimed in one or more of claims 1 to 17, wherein the reaction mixture is extracted with an aqueous solution of a base after decarboxylation, the aqueous phase is separated, the 3-alkoxy-2,4,5-trifluorobenzoic acid is precipitated by acidification, and the precipitate is isolated by filtration.

## Revendications

1. Procédé de préparation d'acides 4-alcoxy-3,5,6-trifluorophtaliques de formule générale 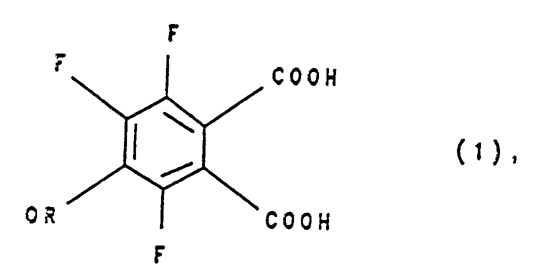 dans laquelle R est un radical alkyle éventuellement une ou plusieurs fois fluoré et ayant de 1 à 5 atomes de carbone, un radical cycloalkyle éventuellement une fois ou plusieurs fois fluoré et ayant de 3 à 5 atomes de car-bone dans le cycle, ou encore un radical araliphatique éven-tuellement une ou plusieurs fois fluoré, et éventuellement d'acides 3-alcoxy-2,4,5-trifluorobenzoïques de formule générale 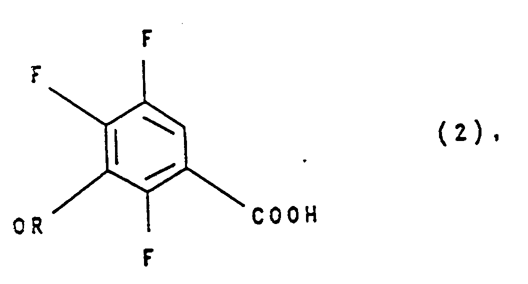 dans laquelle R a les significations ci-dessus, caractérisé en ce gu'on fait réagir dans de l'eau à haute température de l'acide tétrafluorophtalique ou de l'anhydride tétrafluorophtalique avec un alcool de formule générale ROH dans laquelle R a les significations données ci-dessus, et une base soluble dans l'eau, on isole l'acide 4-alcoxy-3,5,6-trifluorophtalique et éventuellement on décarboxyle l'acide 4-alcoxy-3,5,6-trifluorophtalique en présence d'un solvant basique organique et éventuellement d'un solvant inerte à une température de 70 à 180°C, et on isole l'acide 3-alcoxy-2,4,5-trifluorobenzoïque formé.

2. Procédé selon la revendication 1, caractérisé en ce qu'on utilise en tant qu'alcool de formule générale ROH un alcool primaire.

3. Procédé selon la revendication 1 ou 2, caractérisé en ce qu'on utilise en tant qu'alcool de formule ROH le méthanol, l'éthanol, le n-propanol, le n-butanol, le n-pentanol, le cyclopropanol, l'hydroxyméthylcyclopropane, le fluorométhanol, le difluorométhanol, le trifluoro-méthanol, le monofluoréthanol, le difluoréthanol, le trifluoréthanol, le monofluoropropanol, le monofluoro-butanol, le monofluorocyclopropanol, le difluorocyclo-propanol, l'alcool benzylique, l'alcool 4-fluoro-benzylique ou le phényléthanol.

4. Procédé selon l'une ou plusieurs des revendications 1 à 3, caractérisé en ce qu'on utilise en tant qu'alcool de formule ROH le méthanol, l'éthanol, le cyclopropanol, le n-propanol, le fluorométhanol, le difluorométhanol, le trifluorométhanol, le monofluoréthanol, le monofluorobutanol, l'alcool benzylique ou l'alcool 4-fluorobenzylique.

5. Procédé selon l'une ou plusieurs des revendications 1 à 4, caractérisé en ce qu'on utilise de 1 à 100, en particulier de 20 à 80 et de préférence de 40 à 60 moles d'alcool par mole d'acide tétrafluorophtalique ou d'anhydride tétrafluorophtalique.

6. Procédé selon l'une ou plusieurs des revendications 1 à 5, caractérisé en ce qu'on utilise en tant que base soluble dans l'eau un hydroxyde, un carbonate, un phosphate, un hydrogénophosphate ou un dihydrogénophosphate d'un métal alcalin ou d'un métal alcalinoterreux, ou des mélanges de ceux-ci.

7. Procédé selon l'une ou plusieurs des revendications 1 à 6, caractérisé en ce qu'on utilise en tant que base soluble dans l'eau l'hydroxyde de lithium, l'hydroxyde de lithium hydraté, l'hydroxyde de sodium ou l'hydroxyde de potassium.

8. Procédé selon l'une ou plusieurs des revendications 1 à 7, caractérisé en ce qu'on utilise de 1 à 100, en particulier de 1,1 à 10 et de préférence de 2 à 5 moles de base par mole d'acide tétrafluorophtalique ou d'anhydride tétrafluorophtalique.

9. Procédé selon l'une ou plusieurs des revendications 1 à 8, caractérisé en ce qu'on utilise de 1 à 1500, en particulier de 10 à 300 et de préférence de 25 à 200 moles d'eau par mole d'acide tétrafluorophtalique ou d'anhydride tétrafluorophtalique.

10. Procédé selon l'une ou plusieurs des revendications 1 à 9, caractérisé en ce qu'on fait réagir l'acide tétrafluorophtalique ou l'anhydride tétrafluorophtalique avec l'alcool à une température de 25 à 150, en particulier de 50 à 120 et de préférence de 60 à 100°C.

11. Procédé selon l'une ou plusieurs des revendications 1 à 10, caractérisé en ce qu'on chasse par distillation du mélange réactionnel aqueux l'alcool n'ayant pas réagi, et on isole du mélange réactionnel aqueux l'acide 4-alcoxy-3,5,6-trifluorophtalique par addition d'un acide et/ou par extraction.

12. Procédé selon l'une ou plusieurs des revendications 1 à 11, caractérisé en ce qu'on extrait le mélange réactionnel aqueux avec un solvant organique insoluble dans l'eau, en particulier un dialkyléther, ou un ester d'un acide carboxylique aliphatique ayant de 1 à 6 atomes de carbone, ou avec le solvant basique.

13. Procédé selon l'une ou plusieurs des revendications 1 à 12, caractérisé en ce qu'on utilise en tant que solvant basique organique une amine aliphatique à chaîne droite ou ramifiée, un composé éventuellement une ou plusieurs fois alkylé choisi parmi le pyrrole, la pyrrolidone, l'imidazole, l'imidazolidinone, la pyridine, la pyrimidine, la quinoléine, l'isoquinoléine, un carboxa-mide N,N-dialkylé ou des mélanges de ces composés.

14. Procédé selon l'une ou plusieurs des revendications 1 à 13, caractérisé en ce qu'on utilise en tant que solvant basique une trialkylamine ayant de 4 à 20 atomes de carbone dans le résidu alkyle, des mélanges de ces trialkylamines, la collidine, la quinoléine, la N-méthylpyrrolidone, le N,N-diméthylacétamide, la 1,3-diméthyl-imidazolidinone ou des mélanges de ces composés.

15. Procédé selon l'une ou plusieurs des revendications 1 à 14, caractérisé en ce qu'on utilise en tant que solvant inerte des hydrocarbures aromatiques, des hydrocarbures aromatiques une ou plusieurs fois halogénés ou des mélanges de ces derniers.

16. Procédé selon l'une ou plusieurs des revendications 1 à 15, caractérisé en ce qu'on utilise en tant que solvant inerte le toluène, l'éthylbenzène, le mésitylène, l'o-xylène, le m-xylène, le p-xylène, les mélanges techniques de xylènes isomères, le chlorobenzène, le dichlorobenzène, le trichlorobenzène, le chlorotoluène, le dichlorotoluène ou des mélanges de ces derniers.

17. Procédé selon l'une ou plusieurs des revendications 1 à 16, caractérisé en ce qu'on procède à la décarboxylation à une température de 80 à 170°C et en particulier de 100 à 150°C.

18. Procédé selon l'une ou plusieurs des revendications 1 à 17, caractérisé en ce qu'on extrait le mélange réactionnel, après décarboxylation, avec une solution aqueuse d'une base, on sépare la phase aqueuse, on précipite l'acide 3-alcoxy-2,4,5-trifluorobenzoïque par acidification et on l'isole par filtration.
